# EUROPÄISCHE PATENTSCHRIFT

(11) **EP 0 768 996 B1**
(45) Veröffentlichungstag und Bekanntmachung des Hinweises auf die Patenterteilung: **12.05.1999**
(21) Anmeldenummer: 95925765.0
(22) Anmeldetag: 26.06.1995
(51) Int. Cl.: C07C 5/42, C07C 15/46, B01J 23/18

(54) **KATALYSATOR UND VERFAHREN FÜR DIE KATALYTISCHE OXIDATIVE DEHYDRIERUNG VON ALKYLAROMATEN**
CATALYST AND PROCESS FOR THE CATALYTICALLY OXIDATIVE DEHYDROGENATION OF ALKYL AROMATICS
CATALYSEUR ET PROCEDE DE DESHYDROGENATION OXYDATIVE CATALYTIQUE D'AROMATIQUES D'ALKYLE

(30) Priorität: 07.07.1994 DE 4423975
(43) Veröffentlichungstag der Anmeldung: 23.04.1997
(73) Patentinhaber: BASF AKTIENGESELLSCHAFT, 67056 Ludwigshafen (DE)
(72) Erfinder: HAGEMEYER, Alfred, D-67063 Ludwigshafen (DE); LAUTH, Günter, D-23562 Lübeck (DE); LAUTENSACK, Thomas, D-67067 Ludwigshafen (DE); DEIMLING, Axel, D-67434 Neustadt (DE)
(86) Internationale Anmeldenummer: EP9502483
(87) Internationale Veröffentlichungsnummer: WO9601796

(56) Entgegenhaltungen:
- EP-A- 0 403 462
- FR-A- 2 448 519

## Beschreibung

Die Erfindung betrifft ein Verfahren und einen Katalysator für die katalytische oxidative Dehydrierung von Alkylaromaten zu den entsprechenden Alkenylaromaten, vorzugsweise von Ethylbenzol zu Styrol, wobei Wasser gebildet wird und wobei die Dehydrierung in Abwesenheit von freiem (d.h. dem Eduktstrom kontinuierlich zugesetztem) Oxidationsmittel wie molekularem Sauerstoff oder sauerstoffhaltigen Gasen stattfindet und der aus wenigstens einem reduzierbaren Metalloxid bestehende Redoxkatalysator als alleinige Sauerstoffquelle die Funktion eines Sauerstoffspeichers- oder -überträgers übernimmt.

Alkenylbenzole, insbesondere Styrol und Divinylbenzol, stellen wichtige Monomere für technische Kunststoffe dar und werden in großen Mengen produziert. Styrol wird überwiegend durch nicht-oxidative Dehydrierung von Ethylbenzol an einem modifizierten Eisenoxid-Katalysator hergestellt, wobei pro mol Styrol ein mol Wasserstoff entsteht. Nachteiligerweise handelt es sich um eine Gleichgewichtsreaktion, die bei hohen Temperaturen von typischerweise 600 bis 700°C durchgeführt wird und mit einem Umsatz von ca. 60 % bei einer Styrol-Selektivität von etwa 90 % verläuft.

Durch oxidative Dehydrierung, bei der der Eduktstrom mit einem Oxidationsmittel wie molekularem Sauerstoff oder einem sauerstoffhaltigen Gas beaufschlagt wird, läßt sich das Gleichgewicht überwinden und ein fast quantitativer Umsatz erzielen, da als Coprodukt nunmehr Wasser gebildet wird. Auch sind für diese Umsetzung geringere Reaktionstemperaturen erforderlich. Nachteilig an diesem Verfahren ist jedoch die mit der Gegenwart von Sauerstoff einhergehende Selektivitätseinbuße für das Wertprodukt, da die hohe Sauerstoffkonzentration in der Reaktionszone als Nebenreaktion die Totaloxidation begünstigt.

Es ist deshalb vorgeschlagen worden, anstelle von freiem Oxidationsmittel (Sauerstoff) einen aus einem reduzierbaren Metalloxid bestehenden Katalysator als Sauerstoffträger einzusetzen. Dabei wird der Katalysator (gleichzeitig Sauerstoffüberträger) allmählich verbraucht und muß in einem zweiten Schritt regeneriert werden, wodurch die Anfangsaktivität wiederhergestellt wird. In der Regenerierphase können z.B. auch Koksablagerungen abgebrannt werden. Die Regenerierung ist stark exotherm, so daß die freiwerdende Abwärme z.B. für die Erzeugung von Dampf genutzt werden kann.

Durch die Entkopplung von Reduktions- und Oxidationsschritt kann die Selektivität deutlich gesteigert werden.

Für die technische Realisierung dieses Vorschlags gibt es zwei Varianten, nämlich die räumliche und die zeitliche Trennung der beiden Teilschritte.

Bei ersterem wird ein Wanderbett oder eine zirkulierende Wirbelschicht verwendet, so daß die Katalysatorteilchen aus der Dehydrierzone, nach Abtrennung der gebildeten Reaktionsprodukte, zu einem separaten Regenerierungsreaktor gefördert werden, in dem die Reoxidation durchgeführt wird. Der regenerierte Katalysator wird in die Dehydrierzone zurückgeführt. Der Katalysator ist hohen mechanischen Beanspruchungen ausgesetzt und muß daher über eine ausreichende Härte verfügen.

Bei der Ausführung mit einem Festbett wird periodisch zwischen der Eduktzuführung und bei Bedarf nach einer Spülphase dem Regeneriergas umgeschaltet.

Dieses Prinzip der Trennung der zweier Teilschritte der Redoxreaktion unter Einsatz eines reduzierbaren und regenerierbaren Katalysators wurde zuerst für die Oxidation bzw. Ammonoxidation von Propen zu Acrolein und Acrylsäure bzw. Acrylnitril beschrieben (GB 885422; GB 999629; K. Aykan, J. Catal. 12 (1968) 281-190), wobei Arsenat- und Molybdatkatalysatoren verwendet wurden. Der Einsatz des Verfahrens bei der oxidativen Dehydrierung von aliphatischen Alkanen zu Mono- und Diolefinen mit Ferritkatalysatoren (z.B. US 3 440 299, DE 21 18 344, DE 17 93 499) ist ebenfalls bekannt, ebenso wie der Einsatz für die oxidative Kupplung von Methan zu höheren Kohlenwasserstoffen, wobei unterschiedliche Katalysatorklassen zum Einsatz kommen (Z.B. US 4 795 849, DE 3 586 769 mit Mn/Mg/Si-Oxiden; US 4 568 789 mit Ru-Oxid; EP 254 423 mit Mn/B-Oxiden auf MgO; GB 2 156 842 mit Mn₃O₄-Spinellen). Auch die Dehydrodimerisierung von Toluol zu Stilben in Abwesenheit von freiem Sauerstoff mittels reduzierbarer Katalysatoren wie Bi/In/Ag-Oxiden (EP 30 837) ist bekannt. Schließlich wird das Prinzip noch für die Dehydrierung, Dehydrocyclisierung und Dehydroaromatisierung von Paraffinkohlenwasserstoffen zur Benzinveredelung angewandt (US 4 396 537 5 mit Co/P-Oxid-Katalysatoren).

Aus den EP 397 637 und 403 462 ist bekannt, das Verfahrensprinzip für die oxidative Dehydrierung von Paraffinkohlenwasserstoffen und Alkylaromaten einzusetzen. Danach werden reduzierbare Metalloxide verwendet, ausgewählt aus der Gruppe V, Cr, Mn, Fe, Co, Pb, Bi, Mo, U und Sn, aufgebracht auf Trägern aus Tonen, Zeolithen und Oxiden von Ti, Zr, Zn, Th, Mg, Ca, Ba, Si, Al. Besonders bevorzugt ist V/MgO. Die FR-A 2 448 519 beschreibt ein Verfahren zur Dehydrierung von Paraffinen, Monoolefinen und Alkylaromaten an einem Redoxkatalysator, der aus einem mit Molybdänoxid imprägnierten Träger aus Magnesium-silikat, -titanat oder -aluminat besteht.

Obwohl mit diesen Katalysatoren eine hohe Ausbeute erzielt werden kann, kommt es in der Anfangsphase der Dehydrierung, wenn der Kohlenwasserstoff mit dem frisch regenerierten und daher besonders aktiven Katalysator in Kontakt tritt, zu einer sehr starken Vergasung (Totalverbrennung). Abgesehen vom Verlust an Rohstoffen wird dabei auch erheblich mehr Sauerstoff verbraucht als für die bloße Dehydrierung, so daß ein großer Teil des Sauerstoffüberträgers vorzeitig erschöpft ist und sich die Zykluszeiten unnötig verkürzen.

Die Erfindung löst die Aufgabe, ein Verfahren zur Dehydrierung von Alkylaromaten an einem sauerstoffübertragenden Katalysator zu finden, der höhere Umsätze als die nicht-oxidative Dehydrierung ermöglicht und zugleich das nachteilige Verhalten der Katalysatoren der oxidativen Dehydrierung in der Anfangsphase weitgehend vermeidet, so daß die Selektivität erhöht wird und zusätzliche verfahrenstechnische Schritte, z.B. eine partielle Vorreduktion des Sauerstoffüberträgers erspart werden können. Ein weiteres Anliegen der Erfindung ist es, einen besonders harten Katalysator herzustellen, der der mechanischen Beanspruchung in technischen Reaktoren standhält, ohne zu zerfallen.

Es wurde nun ein Verfahren zur katalytischen oxidativen Dehydrierung von Alkylaromaten zu den entsprechenden Alkenylaromaten mittels eines Redoxkatalysators (Sauerstoffüberträgers) gefunden, wobei in einem ersten Reaktionsschritt der Alkylaromat (Edukt) mit einem Katalysator der Grundlage von Bismutoxid mit einem Zusatz von Alkali oder Erdalkali auf einem Träger aus Titanoxid in Abwesenheit von molekularem Sauerstoff unter Reduktion des Katalysators dehydriert und in einem zweiten Reaktionsschritt der reduzierte Katalysator mit einem sauerstoffhaltigen Gas reoxidiert wird. Bi₂O₃ auf einem Titandioxidträger kann als besonders günstiger sauerstoffübertragender Katalysator für die in Abwesenheit von freiem Oxidationsmittel durchgeführte oxidative Dehydrierung verwendet werden. Der Zusatz von Alkali und/oder Erdalkali, bevorzugt Li, Na oder Cs, insbesondere K, unterdrückt die Anfangsvergasung besonders wirksam und ermöglicht hohe Selektivitäts- und damit Ausbeutegewinne an dehydriertem Produkt. Ebenfalls wirksam sind Zusätze von Seltenerdmetallen, wobei Lanthan besonders günstig ist.

Bevorzugte Verfahren können den Unteransprüchen 3 bis 10 entnommen werden.

Ein bevorzugter Katalysator enthält 3 - 30 Gew.-% K₂O, vorzugsweise 5 - 20 Gew.-% K₂O, 5 - 50 Gew.-% Bi₂O₃, vorzugsweise 10 - 30 Gew.-% Bi₂O₃ und TiO₂ in der zur Vervollständigung der Bilanz erforderlichen Menge.

Ein ebenfalls bevorzugter Katalysator enthält 3 - 30 Gew.-% Cs₂O, vorzugsweise 5 - 20 Gew.-% Cs₂O, 5 - 50 Gew.-% Bi₂O₃, vorzugsweise 10 - 30 Gew.-% Bi₂O₃ und TiO₂ in der zur Vervollständigung der Bilanz erforderlichen Menge.

Ein ebenfalls günstiger Katalysator enthält 3 - 40 Gew.-% La₂O₃, vorzugsweise 5 - 30 Gew.-% La₂O₃, 5 - 50 Gew.-% Bi₂O₃, vorzugsweise 10 - 30 Gew.-% Bi₂O₃ und TiO₂ in der zur Vervollständigung der Bilanz erforderlichen Menge.

Ein weiterer günstiger Katalysator enthält 0 - 30 Gew.-% Cs₂O, vorzugsweise 5 - 20 Gew.-% Cs₂O, 0 - 30 Gew.-% K₂O, vorzugsweise 5 - 20 Gew.-% K₂O, 0 - 40 Gew.-% La₂O₃, vorzugsweise 5 - 30 Gew.-% La₂O₃, 5 - 50 Gew.-% Bi₂O₃, vorzugsweise 10 - 30 Gew.-% Bi₂O₃ und TiO₂ in der zur Vervollständigung der Bilanz erforderlichen Menge. Auch die gemeinsame Verwendung z.B. von Cs/La erzielt eine vorteilhafte Wirkung.

Die vorstehenden Mengenverhältnisse beziehen sich auf den fertigen Katalysator in der jeweils beständigsten bzw. der angegebenen Oxidationsstufe. Über die tatsächlichen Bindungsverhältnisse soll damit keine Aussage gemacht und die Erfindung insoweit nicht beschränkt werden; z.B. können sich beim Kalzinieren auch Phasen ausbilden, die höheren Oxidationsstufen des Chroms entsprechen wie Chromate oder Bichromate des Kaliums oder Bismuths.

Der Katalysator kann auf übliche Weise wie Trockenmischen, Aufschlämmen, Imprägnieren, Fällung, Sprühtrocknung etc. hergestellt werden. Die Inhaltsstoffe können z.B. in Form ihrer Oxide, Hydroxide, Carbonate, Acetate, Nitrate oder generell wasserlöslicher Salze mit organischen oder anorganischen Anionen verwendet werden, die beim Erhitzen (Calcinieren) in die entsprechenden Oxide übergehen. Auch Übergangsmetallkomplexe können z.B. verwendet werden. Die Calcinierung erfolgt typischerweise bei Temperaturen oberhalb von 200 (bis 1000)°C, vorzugsweise von 200 bis 800°C und insbesondere von 400 bis 700°C.

Die Dehydrierungsreaktion erfordert eine Temperatur von 200 bis 800°C, bevorzugt 350 bis 550°C und atmosphärischen oder geringfügig erniedrigten oder erhöhten Druck z.B. von 100 mbar bis 10 bar, bevorzugt 500 mbar bis 2 bar, bei einer LHSV von 0,01 bis 20 Stunden⁻¹, bevorzugt 0,1 bis 5 Stunden⁻¹. Neben dem zu dehydrierenden Kohlenwasserstoff können Verdünnungsmittel wie beispielsweise CO₂, N₂, Edelgase oder Dampf zugegen sein. Die Regenerierung des reduzierten Katalysators erfordert Temperaturen zwischen 300 und 900°C, bevorzugt 400 und 800°C, wobei als Oxidationsmittel, z.B. N₂O oder ein sauerstoffhaltiges Gas verwendet wird. Auch hier können Verdünnungsmittel im Reaktorzustrom enthalten sein. Geeignete Regeneriergase sind z.B. Luft, Magerluft oder N₂O-Gemische. Die Regenerierung kann bei vermindertem oder atmosphärischen oder erhöhtem Druck betrieben werden. Bevorzugt sind Drucke im Bereich 500 mbar bis 10 bar.

Bei Verwendung von Lanthan sollte nicht vom Oxid La₂O₃ ausgegangen werden. Statt dessen sollten Verbindungen mit organischen Resten, vorzugsweise La-Acetat, eingesetzt werden, die bei der Calcinierung zu feinverteiltem und oberflächenreichem La₂O₃ führen.

### Beispiel 1

60 g TiO₂ werden mit 33,22 g basischem Bismuthcarbonat Bi₂CO₅ (enthält 81 Gew.-% Bi) und 14,67 g K₂CO₃ trocken gemischt und weiter verfahren wie nachstehend (Vergleichsversuch 2) beschrieben.
Der Katalysator enthält 10 Gew.-% K₂O, 30 Gew.-% Bi₂O₃ und 60 Gew.-% TiO₂.

### Beispiel 2

65 g TiO₂ werden mit 27,69 g basischem Bismuthcarbonat Bi₂CO₅ (enthält 81 Gew.-% Bi) und 14,67 g K₂CO₃ trocken gemischt und weiter wie vorstehend verfahren
Der Katalysator enthält 10 Gew.-% K₂O, 25 Gew.-% Bi₂O₃ und 65 Gew.-% TiO₂.

### Beispiel 3

60 g TiO₂ werden mit 27,69 g basischem Bismuthcarbonat Bi₂CO₅ (enthält 81 Gew.-% Bi) und 22,0 g K₂CO₃ trocken gemischt und weiter wie vorstehend verfahren
Der Katalysator enthält 15 Gew.-% K₂O, 25 Gew.-% Bi₂O₃ und 60 Gew.-% TiO₂.

### Beispiel 4

55 g TiO₂ werden mit 27,69 g basischem Bismuthcarbonat Bi₂CO₅ (enthält 81 Gew.-% Bi) und 29,34 g K₂CO₃ trocken gemischt und weiter wie vorstehend verfahren
Der Katalysator enthält 20 Gew.-% K₂O, 25 Gew.-% Bi₂O₃ und 55 Gew.-% TiO₂.

### Beispiel 5

60 g TiO₂ werden mit 33,2 g basischem Bismuthcarbonat Bi₂CO₅ (enthält 81 Gew.-% Bi) und 11,65 g CsCO₃ trocken gemischt und weiter wie vorstehend verfahren
Der Katalysator enthält 10 Gew.-% Cs₂O, 30 Gew.-% Bi₂O₃ und 60 Gew.-% TiO₂.

### Beispiel 6

60 g TiO₂ werden mit 27,69 g basischem Bismuthcarbonat Bi₂CO₅ (enthält 81 Gew.-% Bi) und 17,34 Cs₂CO₃ trocken gemischt und weiter wie vorstehend verfahren
Der Katalysator enthält 15 Gew.-% Cs₂O, 25 Gew.-% Bi₂O₃ und 60 Gew.-% TiO₂.

### Beispiel 7

55 g TiO₂ werden mit 27,69 g basischem Bismuthcarbonat Bi₂CO₅ (enthält 81 Gew.-% Bi) und 14,67 K₂CO₃ und 11,56 g Cs₂CO₃ trocken gemischt und weiter wie vorstehend verfahren
Der Katalysator enthält 10 Gew.-% Cs₂O, 10 Gew.-% K₂O, 25 Gew.-% Bi₂O₃ und 55 Gew.-% TiO₂.

### Beispiel 8

75 g TiO₂ werden mit 83,05 g basischem Bismuthcarbonat Bi₂CO₅ (enthält 81 Gew.-% Bi), 36,68 g K₂CO₃ und 161,53 g Lanthanacetat C₆H₉LaO₆·aq (enthält 39,56 Gew.-% La) trocken gemischt und weiter wie vorstehend verfahren
Der Katalysator enthält 10 Gew.-% K₂O, 30 Gew.-% La₂O₃, 30 Gew.-% Bi₂O₃ und 30 Gew.-% TiO₂.

### Vergleichsversuch 1

200 g TiO₂ werden mit 129,2 g basischem Bismuthcarbonat Bi₂CO₅ (enthält 81 Gew.-% Bi) trocken gemischt und weiter nach Vergleichsversuch 2 verfahren. Der Katalysator enthält 63 Gew-% TiO₂ und 37 Gew.-% Bi₂O₃.

### Vergleichsversuch 2

200 g TiO₂ werden mit 58,6 g NH₄VO₃ trocken gemischt und weiter nach Vergleichsbeispiel 2 verfahren.

Der Katalysator enthält 19 Gew.-% V₂O₅ und 81 Gew.-% TiO₂.

### Vergleichsversuch 3 (nach EP 397637)

In 8 1 Wasser werden 336,3 g Ammonium-meta-vanadat und 900 g Magnesiumoxid eingerührt und danach 1 h kräftig gerührt. Anschließend wird am Sprühtrockner versprüht. Das erhaltene Sprühpulver wird für 2 h im Kneter behandelt, wobei wenig Wasser und Verstrangungshilfsmittel zur Knetmasse untergeknetet werden. Anschließend wird die Knetmasse mittels einer Strangpresse zu 3 mm Vollsträngen verformt. Die Stränge werden für 16 h bei 120°C getrocknet und danach für 4 h bei 600°C calciniert. Man erhält einheitlich gelb-gefärbte Stränge mit geringer Härte. Für die Reaktorversuche wird eine 0,05 bis 0,1 mm Splittfraktion ausgesiebt. Der Katalysator enthält 22,5 Gew.-% V₂O₅ und 77,5 Gew.-% MgO.

### Vergleichsversuch 4

100 g MgO-Pulver und 58,1 g NH₄VO₃-Pulver werden für 1 h trocken gemischt. Anschließend wird die Mischung im Kneter für 2,5 h geknetet. Danach wird die Knetmasse mittels einer Strangpresse zu 3 mm Vollsträngen verformt. Die Stränge werden für 2 h bei 120°C getrocknet. Anschließend wird bei 500°C für 2 h calciniert. Für die Reaktorversuche wird eine 0,05 bis 0,1 mm Splittfraktion ausgesiebt. XRD zeigt nur die Linien von V₂O₅ und MgO. Der Katalysator enthält 31 Gew.-% V₂O₅ und 69 Gew.-% MgO.

### Vergleichsversuch 5

100 g TiO₂-Pulver (DT-51 von BASF AG) werden vorgelegt und weiter nach Vergleichsversuch 2 verfahren. Der Katalysator besteht aus reinem TiO₂.

### Vergleichsversuch 6

276,85 g basisches Bismuthcarbonat werden vorgelegt und weiter nach Vergleichsversuch verfahren. Der Katalysator besteht aus reinem Bi₂O₃.

### Leistungstests

Die Schnitthärte der Katalysatoren wird am 3 mm Vollstrang ermittelt, indem die zum Durchschneiden des Stranges mit einem scharfen Messer (Klingenbreite 0,6 mm) erforderliche Kraft in N gemessen wird.

Die katalytisch-oxidative Dehydrierung von Ethylbenzol zu Styrol wird in einem Pulsreaktor bei einer Temperatur von 500°C durchgeführt. Dabei wird ein Mikrofestbett (Einwaage Katalysator: 0,3 g) pulsierend mit reinem Ethylbenzol beaufschlagt und die entstandenen Reaktionsprodukte für jeden Puls quantitativ gaschromatographisch erfaßt. Zwischen zwei aufeinanderfolgenden Ethylbenzol-Pulsen (ca. 1,5 min) strömt Helium durch den Reaktor. Ein einzelner Puls enthält 380 mg Ethylbenzol. Die Strömungsgeschwindigkeit des Trägergases beträgt 21,5 ml/min. Auf diese Weise läßt sich das Zeitverhalten des Katalysators ohne Totzeiten von Anfang an mit hoher Zeitauflösung verfolgen.

Zu Beginn der Reaktion ist der Katalysator hochaktiv, so daß hohe, nahezu quantitative Umsätze von Ethylbenzol beobachtet werden. Im weiteren Verlauf der Reaktion verbessert sich die Selektivität zu Styrol stetig bis auf einen Endwert. Mit fortschreitender Versuchsdauer wird jedoch der Katalysator in dem Maße, wie sein Sauerstoffgehalt aufgezehrt wird, mehr und mehr deaktiviert, so daß der Umsatz sinkt. Je nach Katalysator wird nach 90 bis 200 Pulsen regeneriert. Die Styrolausbeute als Produkt aus Selektivität und Umsatz durchläuft i.a. ein flaches Maximum. Die in der Tabelle aufgelistete Ausbeute bezieht sich auf diesen Maximalwert.

Nach Beendigung der Dehydrierreaktion wird auf einen Luftstrom von 25 ml/min umgeschaltet und der Katalysator für ca. 1 h bei 500°C regeneriert. Danach schließt sich der nächste Zyklus an. Es werden jeweils mehrere Zyklen untersucht.

Die Ergebnisse der Beispiele/Versuche sind in der nachstehenden Tabelle wiedergegeben. Die Tabelle enthält eine Zusammenfassung der hergestellten Katalysatoren, der relativen Massenverhältnisse der Komponenten, der Schnitthärte und der Ergebnisse der Tests (Mittelwerte aus mehreren Versuchen) zur Dehydrierung von Ethylbenzol in einem Festbettreaktor.

Aus der Tabelle können folgende Schlußfolgerungen abgeleitet werden:

Die Systeme entsprechend dem Stand der Technik sind sehr aktiv und erlauben eine hohe maximale Styrolausbeute. Der entscheidende Nachteil besteht in der starken Anfangsvergasung, was zu enormen Verlusten an Ethylbenzol führt und zu Lasten des Sauerstoffreservoirs des Katalysators geht. Insbesondere werden die ersten Ethylbenzol-Pulse vollständig verbrannt (100 % Vergasung zu wertlosem Kohlendioxid), so daß die rechnerische Anfangsselektivität für Styrol über die ersten Pulse hinweg bei Null liegt.

Demgegenüber zeichnen sich die erfindungsgemäßen Systeme bei ebenfalls sehr hoher Aktivität durch deutlich geringere Vergasung aus. So beträgt die Anfangsvergasung (1. Puls) nur 30 Gew.-%, verglichen mit 100 Gew.-% nach dem Stand der Technik.

Die Styrolausbeute liegt deutlich oberhalb derer, die mittels nichtoxidativer Dehydrierung erzielt werden können, und zwar bei abgesenkter Reaktionstemperatur.

Wie Vergleichsversuch 1 und 2 zeigt, ist bei den bekannten Katalysatoren die Präparationsmethode von Bedeutung. Es wird ersichtlich, daß der bekannte sprühgetrocknete Katalysator deutlich besser ist als der trocken gemischte.

Der Verfahrensschritt der Sprühtrocknung bei der Katalysatorherstellung bedeutet nun aber einen zeit- und energieintensiven Verfahrensschritt, der höhere Herstellungskosten zur Folge hat. Bei der Sprühtrocknung von MgO muß eine größere Wassermenge je Gramm Feststoff zugesetzt werden als bei TiO₂, was zur Folge hat, daß dieser Verfahrensschritt mit TiO₂ schneller vonstatten geht als mit MgO.

Die Styrolselektivität des erfindungsgemäßen Katalysators im Maximum ist vergleichbar mit dem Stand der Technik, wobei die Katalysatorpräparation durch Trockenmischen einen deutlich geringeren zeitlichen und apparativen Aufwand erfordert.

Der erfindungsgemäße Katalysator erzeugt zwar in der Anfangsphase (statt Gas, wie beim Stand der Technik) leicht erhöhte Mengen der Nebenprodukte Benzol und Toluol, die dann aber mit fortschreitender Reaktionszeit rasch abnehmen. Die Bildung von Benzol und Toluol ist unproblematisch im Vergleich zur CO₂-Bildung, da Toluol ein verkaufsfähiges Produkt ist und Benzol in die Ethylbenzolherstellung zurückgeführt werden kann und beide damit nicht verloren sind. Der erfindungsgemäße Katalysator ist also auch in dieser Hinsicht dem Stand der Technik überlegen, unabhängig davon, daß die mittlere Styrolausbeute bzw. die Gesamtstyrolausbeute günstiger ist als bei den bekannten Katalysatoren.

Der entscheidende Vorteil des erfindungsgemäßen Systems ist die deutlich reduzierte Anfangsvergasung, die gegenüber dem Stand der Technik enorme Gewinne in der Anfangsstyrolselektivität ermöglicht.

Der erfindungsgemäße Katalysator ist im übrigen besonders abriebfest. Dies hat Vorteile bei der mechanischen Handhabung der Katalysatoren (Transport, Ein- und Ausbau im Reaktor) und der mechanischen Belastung der Katalysatoren in der Festbettschüttung, wobei auch berücksichtigt werden muß, daß bei der Reoxidation erhebliche Wärmemengen frei werden, die den Katalysator mechanisch stark beanspruchen. Bei gleicher Präparationstechnik weist der erfindungsgemäße Katalysator (Beispiel 1 bis 7) deutlich bessere Werte für die mechanische Festigkeit gegenüber den Vergleichsversuchen 1 und 2 auf.

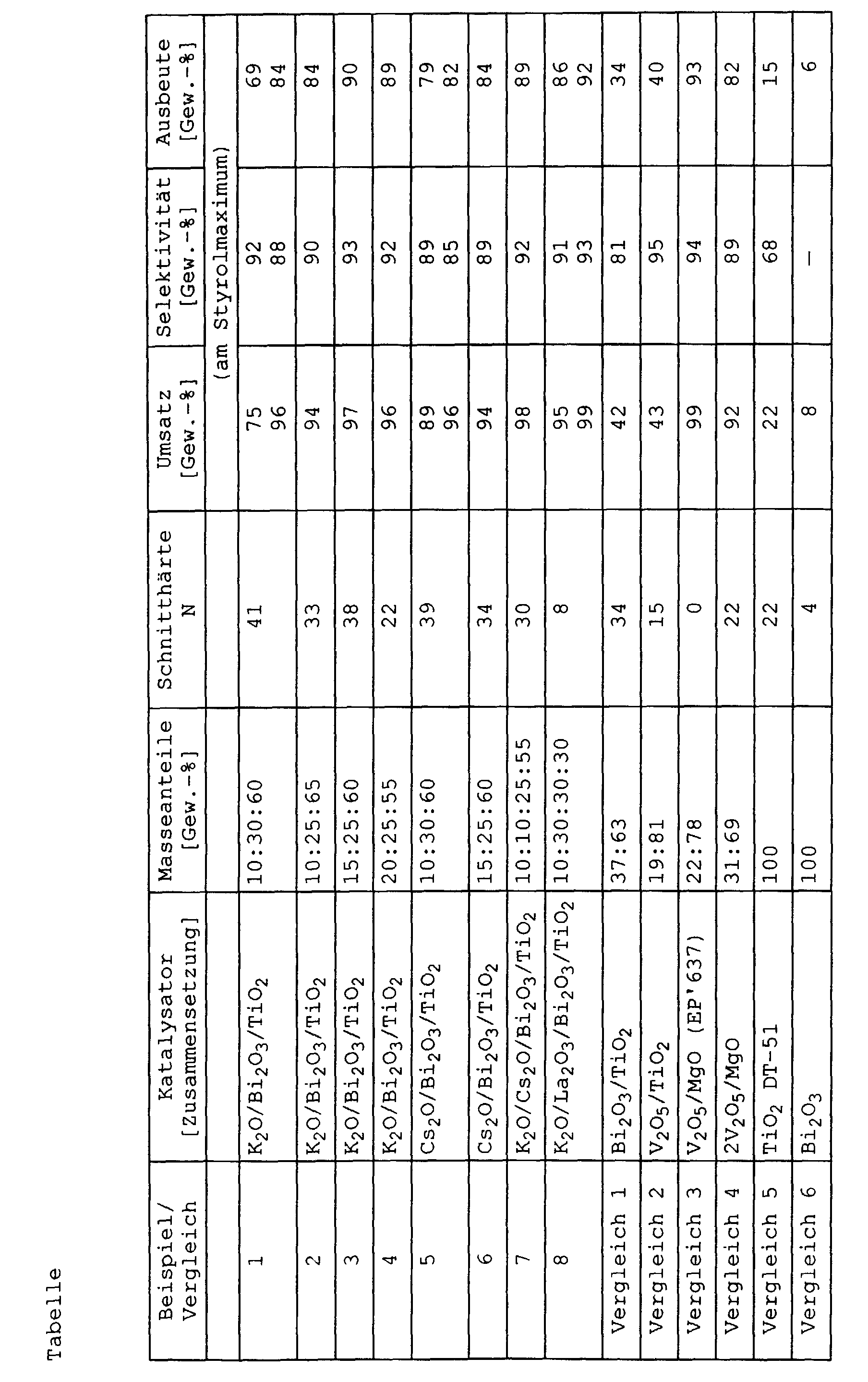

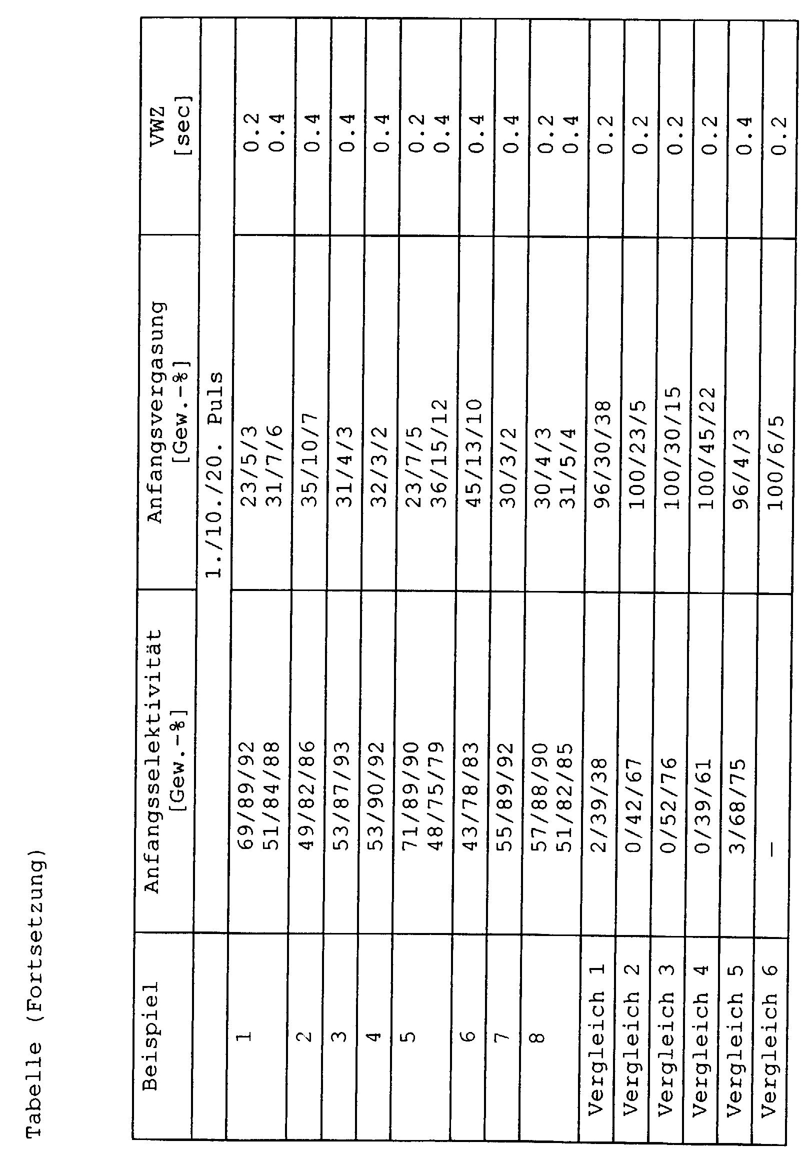

## Patentansprüche

1. Verfahren zur katalytischen oxidativen Dehydrierung von Alkylaromaten zu den entsprechenden Alkenylaromaten mittels eines Redoxkatalysators (Sauerstoffüberträgers), dadurch gekennzeichnet, daß in einem ersten Reaktionsschritt der Alkylaromat (Edukt) mit einem Katalysator auf der Grundlage von Bismutoxid mit einem Zusatz von Alkali oder Erdalkali auf einem Träger aus Titanoxid in Abwesenheit von molekularem Sauerstoff unter Reduktion des Katalysators dehydriert und in einem zweiten Reaktionsschritt der reduzierte Katalysator mit einem sauerstoffhaltigen Gas reoxidiert wird.

2. Verfahren nach Anspruch 1, dadurch gekennzeichnet, daß der Katalysator außerdem Lanthan enthält.

3. Verfahren nach Anspruch 1, dadurch gekennzeichnet, daß der Katalysator ein anorganisches Bindemittel wie Aluminiumoxid enthält.

4. Verfahren nach Anspruch 1, dadurch gekennzeichnet, daß der erste und der zweite Reaktionsschritt zeitlich oder räumlich abwechselnd stattfinden und der Katalysator fest angeordnet ist.

5. Verfahren nach Anspruch 2, dadurch gekennzeichnet, daß die Entkopplung der Teilschritte zeitlich durch periodisches Umschalten des Reaktoreingangsstromes zwischen Edukt und Oxidationsmittel bewirkt wird.

6. Verfahren nach Anspruch 2, dadurch gekennzeichnet, daß der Katalysator fest angeordnet ist und zwischen den Teilschritten eine Spülphase eingefügt ist, in der der Festbettreaktor von einem Spülgas durchströmt wird.

7. Verfahren nach Anspruch 5, dadurch gekennzeichnet, daß als Spülgas CO₂, N₂, H₂O oder Edelgas eingesetzt wird.

8. Verfahren nach Anspruch 2, dadurch gekennzeichnet, daß die räumliche Entkopplung der Teilschritte unter Verwendung einer zirkulierenden Wirbelschicht bewirkt wird, indem Katalysatorteilchen zyklisch zwischen einem Dehydrierreaktor und einem Regenerierungsreaktor im Kreis geführt werden.

9. Verfahren nach Anspruch 1, dadurch gekennzeichnet, daß Ethylbenzol zu Styrol dehydriert wird.

10. Verfahren nach Anspruch 1, wobei die Dehydrierung im Temperaturbereich zwischen 200 und 800°C bei einem Druck von 100 mbar bis 10 bar mit einer Flüssig-Raumgeschwindigkeit (liquid hourly space velocity; LHSV) von 0.01 bis 20 Stunden-1 durchgeführt wird.

11. Katalysator zur Durchführung des Verfahrens nach Anspruch 1, bestehend im wesentlichen aus 5 bis 50 Gew.-% Bismut(3)-Oxid, 3 bis 30 Gew.-% K₂O oder Cs₂O und im übrigen Titan-Oxid mit der Maßgabe, daß die Summe der Gewichtsprozente 100 ergibt, sowie weitere, im wesentlichen unwirksame Bestandteile in untergeordneter Menge.

12. Katalysator nach Anspruch 11, enthaltend ferner 5 bis 30 Gew.-% Lanthanoxid mit der Maßgabe, daß die Summe der Gewichtsprozente 100 ergibt, sowie weitere, im wesentlichen unwirksame Bestandteile in untergeordneter Menge.

## Claims

1. A process for the catalytic oxidative dehydrogenation of alkylaromatics to give the corresponding alkenylaromatics by means of a redox catalyst (oxygen carrier), wherein, in a first reaction step, the alkylaromatic (starting material) is dehydrogenated with a catalyst based on bismuth oxide with the addition of alkali metal or alkaline earth metal on a carrier consisting of titanium oxide in the absence of molecular oxygen with reduction of the catalyst and, in a second reaction step, the reduced catalyst is reoxidized with an oxygen-containing gas.

2. A process as claimed in claim 1, wherein the catalyst additionally contains lanthanum.

3. A process as claimed in claim 1, wherein the catalyst contains an inorganic binder such as alumina.

4. A process as claimed in claim 1, wherein the first and the second reaction steps take place alternately in terms of time or at alternate places and the catalyst is arranged as a fixed bed.

5. A process as claimed in claim 2, wherein decoupling of the steps is effected by periodically switching the reactor inlet stream between starting material and oxidizing agent.

6. A process as claimed in claim 2, wherein the catalyst is arranged as a fixed bed and a flushing phase in which a flushing gas flows through the fixed-bed reactor is introduced between the steps.

7. A process as claimed in claim 5, wherein the flushing gas used is CO₂, N₂, H₂O or a noble gas.

8. A process as claimed in claim 2, wherein the spatial decoupling of the steps is effected with the use of a circulating fluidized bed, by circulating catalyst particles cyclically between a dehydrogenation reactor and a regeneration reactor.

9. A process as claimed in claim 1, wherein ethylbenzene is dehydrogenated to styrene.

10. A process as claimed in claim 1, wherein the dehydrogenation is carried out at from 200 to 800°C and at from 100 mbar to 10 bar at a liquid hourly space velocity (LHSV) of from 0.01 to 20 h⁻¹.

11. A catalyst for carrying out the process as claimed in claim 1, consisting essentially of from 5 to 50% by weight of bismuth(3) oxide and from 3 to 30% by weight of K₂O or Cs₂O, the remainder being titanium oxide, with the proviso that the sum of the percentages by weight is 100, and further, essentially inactive components in a minor amount.

12. A catalyst as claimed in claim 11, further containing from 5 to 30% by weight of lanthanum oxide with the proviso that the sum of the percentages by weight is 100, and further, essentially inactive components in a minor amount.

## Revendications

1. Procédé de déshydrogénation oxydative catalytique de composés alkylaromatiques en composés alcénylaromatiques correspondants au moyen d'un catalyseur rédox (agent de transfert d'oxygène), caractérisé en ce que, dans une première étape de réaction, le composé alkylaromatique (éduit) est déshydrogéné avec un catalyseur à base d'oxyde de bismuth avec une addition d'un métal alcalin ou alcalino-terreux sur un support en dioxyde de titane en absence d'oxygène moléculaire par réduction du catalyseur et en ce que, dans une deuxième étape de réaction, le catalyseur réduit est réoxydé avec un gaz contenant de l'oxygène.

2. Procédé selon la revendication 1, caractérisé en ce que le catalyseur contient en outre du lanthane.

3. Procédé selon la revendication 1, caractérisé en ce que le catalyseur contient un agent liant inorganique, tel que de l'oxyde d'aluminium.

4. Procédé selon la revendication 1, caractérisé en ce que la première et la deuxième étape de réaction alternent dans le temps ou dans l'espace et en ce que le catalyseur est disposé sous forme fixée.

5. Procédé selon la revendication 2, caractérisé en ce que le découplage des étapes partielles est réalisé dans le temps par commutation périodique du flux à l'entrée du réacteur entre l'éduit et l'agent oxydant.

6. Procédé selon la revendication 2, caractérisé en ce que le catalyseur est disposé sous forme fixée et en ce qu'on insère entre les étapes partielles une phase de rinçage, au cours de laquelle le réacteur à lit fixe est traversé par un gaz de rinçage.

7. Procédé selon la revendication 5, caractérisé en ce qu'on utilise à titre de gaz de rinçage du CO₂, du N₂, du H₂O ou un gaz noble.

8. Procédé selon la revendication 2, caractérisé en ce que le découplage dans l'espace des étapes partielles est réalisé en utilisant une couche fluidisée circulante, en faisant circuler les particules de catalyseur de façon cyclique entre un réacteur de déshydrogénation et un réacteur de régénération.

9. Procédé selon la revendication 1, caractérisé en ce que de l'éthylbenzène est déshydrogéné en styrène.

10. Procédé selon la revendication 1, dans lequel la déshydrogénation est effectuée dans la gamme de températures entre 200 et 800 °C à une pression de 100 mbar à 10 bar avec une vitesse spatiale liquide (liquid hourly space velocity ; LHSV) de 0,01 à 20 h⁻¹.

11. Catalyseur pour la réalisation du procédé selon la revendication 1, constitué essentiellement de 5 à 50 % en poids d'oxyde de bismuth (3), de 3 à 30 % en poids de K₂O ou de Cs₂O et, pour le reste, d'oxyde de titane, sous condition que la somme des pourcentages en poids vaut 100, de même que d'autres constituants essentiellement inactifs en quantités secondaires.

12. Catalyseur selon la revendication 11, contenant en outre 5 à 30 % en poids d'oxyde de lanthane sous condition que la somme des pourcentages en poids vaut 100, de même que d'autres constituants essentiellement inactifs en quantités secondaires.
